# EUROPÄISCHE PATENTSCHRIFT

(11) **EP 2 624 788 B1**
(45) Veröffentlichungstag und Bekanntmachung des Hinweises auf die Patenterteilung: **03.01.2018**
(21) Anmeldenummer: 11749206.6
(22) Anmeldetag: 31.08.2011
(51) Int. Cl.: A61F 2/34, A61F 2/30, A61F 2/00

(54) **GELENKPFANNE UND VERFAHREN ZUR HERSTELLUNG EINER GELENKPFANNE**
JOINT SOCKET AND METHOD FOR PRODUCING A JOINT SOCKET
CAVITÉ COTYLOÏDE ET PROCÉDÉ DE FABRICATION D'UNE CAVITÉ COTYLOÏDE

(30) Priorität: 07.10.2010 CH 16402010
(43) Veröffentlichungstag der Anmeldung: 14.08.2013
(73) Patentinhaber: Jossi Holding AG, 8546 Islikon (CH)
(72) Erfinder: MEYENHOFER, Andreas, CH-8255 Schlattingen (CH); SCHMIDT, Martin, CH-9545 Wängi (CH); GUGLER, Christian, CH-8500 Frauenfeld (CH); NADLER, Daniel, CH-8442 Hettlingen (CH)
(74) Vertreter: Hepp Wenger Ryffel AG
(86) Internationale Anmeldenummer: PCT/EP2011/065016
(87) Internationale Veröffentlichungsnummer: WO 2012/045530

(56) Entgegenhaltungen:
- EP-A1- 1 290 992
- EP-A2- 2 008 619
- WO-A1-99/60955
- WO-A2-2005/087141
- US-A1- 2007 239 283
- US-A1- 2008 077 249
- US-A1- 2009 005 879
- US-B1- 6 488 713

## Beschreibung

Die vorliegende Erfindung betrifft eine Gelenkpfanne gemäss dem Oberbegriff des Anspruchs 1 sowie ein Verfahren zur Herstellung einer Gelenkpfanne gemäss Anspruch 9. Gelenkpfannen bestehen in den meisten Fällen aus einer Innenschale, welche zur Aufnahme eines entsprechenden Gelenkkopfes geeignet ist und einer äusseren Schale, welche zur Verankerung in den Knochen dient. Die Innenschale besteht dabei aus tribologisch geeigneten Materialen, wie bestimmten Metalllegierungen, Polyethylen oder Keramiken, während die Aussenschicht meist aus einem osteointegrierbarem Metall oder einer Metalllegierung besteht. Die beiden Schalen werden dabei entweder bei der Herstellung miteinander verbunden (nicht-modulare Pfannen) oder das Zusammensetzen erfolgt während der Operation (modulare Pfannen). Bei nicht-modularen Pfannen kann die Innenschale aus einem Kunststoff, wie Polyethylen, Polyoxyethylen (POM), Polyetheretherketon (PEEK) oder Polyurethan (PU) beispielsweise direkt in eine metallische Aussenschale eingefügt oder eingeklebt werden. Alternativ können die Schalen auch mittels Formschluss miteinander verbunden werden.
Die EP 2 008 619 beschreibt eine Gelenkpfanne, bei der die Innen- und die Aussenschale mittels Presssitz verbunden sind. Als mögliches Verfahren wird das Aufschrumpfen der äusseren Metallschale auf die keramische Innenschale erwähnt.
In der US 6,488,713 wird ein Verfahren beschrieben, um bei einer modularen Gelenkpfanne eine Innenschale aus Polyethylen mit einer metallischen Aussenschale zu verbinden. Dabei wird die Innenschale durch Verwendung von flüssigem Stickstoff in die Aussenschale eingedehnt.

Nachteilig bei diesen Verfahren und Gelenkpfannen ist, dass die Kontaktfläche zwischen der Aussen- und der Innenschale nur geringfügig nach aussen abgedichtet wird. Dadurch können Körperflüssigkeiten zwischen die Schalen gelangen und dadurch Abriebpartikel, die beispielsweise beim Einsetzen oder während dem Gebrauch des Gelenks entstehen, daraus austreten. Weiter können kleine, nicht hermetisch abgedichtete Volumina mit geringem Ausstausch mit der Umgebung zur Keimbildung oder zur Spaltkorossion führen (Abfall des lokalen pH-Wertes).

Die US 2008/0077249 A1 beschreibt eine Gelenkpfanne umfassend eine Aussen- und eine Innenschale, wobei die Innenschale gekühlt in die Aussenschale eingesetzt wird. Die Innenschale weist ein umlaufendes Rückhaltelement (Rippe) auf, welches in ein korrespondierendes Element (Nut) der Aussenschale greift. Auf diese Weise bilden Kontaktflächen der Aussen- und der Innenschale eine dichtende Wirkung aus. Nachteilig an dieser Anordnung ist, dass Flüssigkeiten zwischen die Aussenschale und die Innenschale bis hin zu den die dichtende Wirkung vermittelnden Kontaktflächen gelangen können.

Die Aufgabe der vorliegenden Erfindung ist es demnach, die Nachteile der bekannten Gelenkpfannen zu umgehen und insbesondere, eine Gelenkpfanne zu schaffen, bei der die beiden Schalen einen sicheren Sitz zueinander aufweisen und bei denen die Kontaktfläche zwischen den Schalen gegen Aussen und Innen abgedichtet ist. Diese Aufgabe wird mit einer Gelenkpfanne gemäss Anspruch 1 gelöst.

Die erfindungsgemässe Gelenkpfanne für eine Hüftgelenksprothese besteht mindestens aus einer metallischen Aussenschale und einer vorzugsweise keramischen Innenschale. Die Aussenschale und die Innenschale werden mittels Schrumpfsitz kraftschlüssig zusammengehalten und durch mindestens ein zusätzliches formschlüssiges Mittel miteinander verbunden. Das mindestens eine formschlüssige Mittel ist dabei im äquatorialen Randbereich der Gelenkpfanne derart umlaufend angeordnet, dass dieses durch die Schrumpfspannung eine dichtende Wirkverbindung zwischen Aussenschale und Innenschale bildet.

Dabei weist die Aussenschale einen umlaufenden Kragen auf, welcher über den unteren Rand der Innenschale greift. Der Kragen kann dabei derart beschaffen sein, dass dieser das Aufsetzen der erhitzten Aussenschale auf die Innenschale nicht behindert. Beim anschliessenden Erkalten und Schrumpfen der Aussenschale greift der Kragen in den unteren Rand der Innenschale ein.

Durch die erfindungsgemässe Kombination von Schrumpfsitz und formschlüssigem Mittel wird ein sicherer Sitz der Aussenschale auf der Innenschale erreicht. Zudem verhindert der Kraftschluss relative Bewegungen der beiden Schalen zueinander.

Die auftretende Schrumpfspannung drückt die Formschlussmittel ineinander. Dies führt nicht nur zu einem besseren Sitz der Formschlussmittel zueinander, sondern führt gleichzeitig auch dazu, dass zwischen den Formschlussmitteln eine zusätzliche Dichtwirkung auftritt. Da die Formschlussmittel im äquatorialen Randbereich der Gelenkpfanne angeordnet sind, wird die Grenzfläche zwischen den beiden Schalen gegen aussen hin abgedichtet. Dies verhindert einerseits das Eindringen von Körperflüssigkeiten, wie beispielsweise Blut zwischen die Schalen. Andererseits wird das Austreten von Abriebpartikeln, welche zwischen den Schalen beim operativen Einsetzen der Pfanne oder durch Gebrauch des Implantats entstehen können, verhindert.

Das Aufschrumpfen erfolgt durch Erhitzen der metallischen Aussenschale mit anschliessendem Aufbringen derselben auf die vorzugsweise keramische Innenschale. Beim Erkalten schrumpft die Aussenschale und verbindet sich dabei kraftschlüssig mit der Innenschale. Durch das Erhitzen werden zusätzlich allfällig vorhandene Keime abgetötet, wobei zwischen den Schalen ein keimfreier Raum entsteht.

Das formschlüssige Mittel umfasst bevorzugterweise mindestens eine umlaufende Rippe oder Lippe, welche bevorzugt in eine umlaufende Nut eingreift.

Die mindestens eine umlaufende Rippe kann dabei auf der Innenseite der Aussenschale angebracht sein. Alternativ kann die mindestens eine Rippe aber auch auf der Aussenseite der Innenschale umlaufend angebracht sein. Die mindestens eine Rippe kann dabei jedes geeignete Profil aufweisen, wie beispielsweise kalotten- oder quaderförmig. Besonders bevorzugt weist die Rippe ein dreieckiges oder keilförmiges Profil auf.

Bevorzugterweise greift die umlaufende Rippe in eine umlaufende Nut ein. Die Nut ist dabei auf der jeweiligen anderen Schale derart angeordnet, dass die Rippe beim Aufschrumpfen passend oder mit leichtem Übermass in diese eingreifen kann. Durch die Schrumpfspannung wird die Rippe in die Nut gedrückt, wobei eine Dichtwirkung entsteht. Alternativ können die beiden Schalen auch derart beschaffen sein, dass die Formschlusselemente beim Aufsetzen der erhitzten Aussenschale zunächst ineinander schnappen. Anschliessend bewirkt die Schrumpfspannung, dass die Formschlusselemente derart ineinander gedrückt werden, dass durch die zusätzliche plastische Verformung der Formschlusselemente eine dichtende Wirkung entsteht.

Als formschlüssige Mittel kann die Gelenkpfanne ferner auch mehrere umlaufende Rippen umfassen, welche bevorzugt in umlaufende Nuten eingreifen. Dabei können alle Rippen auf der gleichen Schale angeordnet sein. Alternativ können auf einer Schale auch eine oder mehrere Rippen und eine oder mehrere Nuten angeordnet sein.

In einer bevorzugten Ausführungsform ist ein zusätzliches Dichtelement zwischen einer Rippe und der komplementären Nut angeordnet. Durch die Schrumpfspannung wird die Rippe in das Dichtelement gedrückt, was die Dichtwirkung des Formschlusses erhöht. Das zusätzliche Dichtelement ist bevorzugt als Dichtring ausgestaltet. Das zusätzliche Dichtelement kann aus jedem geeigneten Werkstoff bestehen, beispielsweise aus einem Kunststoff wie Polyethylen oder aber auch aus einem metallischen Werkstoff.

Die Gelenkpfanne kann alternativ zwischen der Aussen- und der Innenschale mindestens ein zusätzliches, umlaufendes Dichtelement aufweisen. Das zusätzliche Dichtelement ist dabei ausserhalb der formschlüssigen Mittel angeordnet. Bevorzugt ist das zusätzliche Dichtelement als Dichtring ausgestaltet. Alternativ kann das zusätzliche Dichtelement auch als Dichtlippe ausgestaltet sein, welche durch die Schrumpfspannung gegen eine der Schalen gedrückt wird. Je nach Ausgestaltung kann sich die Dichtlippe dabei verbiegen oder geringfügig in die Schale eindringen. Dadurch ergibt sich ein zusätzlicher Dichteffekt.

Die metallische Aussenschale besteht aus oder umfasst Titan, eine Titanlegierung, eine Stahllegierung oder eine Cobalt-Chrom-Legierung. Die Aussenschale kann dabei auf ihrer Aussenseite über Verankerungselemente verfügen, um die Befestigung im Knochen zu begünstigen. Eine Vielzahl unterschiedlicher Ausführungsformen derartiger Verankerungselemente sind dem Fachmann bekannt. Ferner kann die Aussenschale auch eine Beschichtung aufweisen.

Die Innenschale besteht aus oder umfasst bevorzugterweise einen keramischen Werkstoff. Bevorzugte Keramiken sind dabei Aluminiumoxidkeramiken, Zirkonoxidkeramiken, Aluminium-Zirkon-Mischkeramiken, wie beispielsweise aluminium- toughened zirconia (ATZ) oder zirconia- toughened aluminia (ZTA), sowie Karbidkeramiken und Nitridkeramiken. Alternativ kann die Innenschale auch aus einer Cobaltbasislegierung oder aus einer Eisenbasislegierung bestehen. Der Werkstoff sollte dabei jedoch stets geeignete tribologische Eigenschaften sowie eine hohe Abriebfestigkeit aufweisen, um als Gleitfläche für die Gelenkkugel einer Prothese zu dienen.

Alternativ kann das mindestens eine formschlüssiges Mittel eine Schneidkante auf der einen Schale umfassen, welche infolge der Schrumpfspannung in die andere Schale gräbt. Bei dieser Ausführungsform muss nur eine der beiden Schalen mit einem Formschlusselement ausgestattet werden, da sich das komplementäre Element durch das Eingraben der Schneidkante ergibt. Dies führt zu einer einfacheren Herstellung der erfindungsgemässen Gelenkspfanne sowie einer höheren Dicht- und Arretierwirkung zwischen den Schalen.

Bevorzugt ist die Schneidkante dabei auf derjenigen Schale angeordnet, deren Material gegenüber dem Material der anderen Schale einen höheren Härtegrad aufweist. Dadurch kann sich die Schneidkante infolge der Schrumpfspannung in die andere Schale eingraben. Bevorzugterweise besteht die Aussenschale dabei aus einem metallischen Werkstoff und die Innenschale aus einem keramischen oder einem polymeren Werkstoff. Dabei kann der keramische Werkstoff einen höheren Härtegrad aufweisen, als der metallische oder polymere Werkstoff. Die entsprechenden Materialeigenschaften und vorteilhaften Materialpaarungen sind dem Fachmann bekannt und werden hier nicht näher beschrieben.

Alternativ kann auch auf der aus dem weicheren Werkstoff bestehenden Schale eine Rippe oder Lippe angebracht sein, welche beim Aufschrumpfen durch die Schrumpfspannung an die Schale aus dem härteren Werkstoff gedrückt wird. Dadurch verformt sich diese Rippe oder Lippe, wobei eine Dichtwirkung entsteht. Bei dieser Ausführungsform kann die Rippe oder Lippe auch in eine in der Schale aus härterem Werkstoff umlaufend angebrachte Nut gedrückt werden.

Der härtere Werkstoff weist dabei eine Härte von bevorzugt 1200 bis 2200 HV auf, während das weichere Material eine Härte von zwischen 300-400 HV aufweist.

Bevorzugt wird die erfindungsgemässe Gelenkpfanne als Teil einer Hüftprothese verwendet. Alternativ kann die erfindungsgemässe Gelenkpfanne auch in anderen Prothesen verwendet werden, wie beispielsweise für die Schulter oder für Sprunggelenke.

Eine weitere Aufgabe der vorliegenden Erfindung besteht darin, ein geeignetes Verfahren vorzulegen, welches die Herstellung einer Gelenkpfanne ermöglicht, welche über einen guten Sitz der Aussenschale auf der Innenschale verfügt und bei der die Kontaktfläche zwischen den Schalen gegen Aussen und Innen hin abgedichtet ist. Diese Aufgabe wird mit einem Verfahren nach Anspruch 9 gelöst.

Beim erfindungsgemässen Verfahren wird eine metallische Aussenschale auf eine bevorzugt keramische Innenschale derart aufgeschrumpft, dass eine kraftschlüssige Verbindung entsteht, wobei mindestens ein umlaufendes formschlüssiges Mittel als umlaufender Kragen im äquatorialen Randbereich der Aussenschale über den unteren Rand der Innenschale dichtend greift.

Durch das zusätzliche Eingreifen mindestens eines umlaufenden Formschlussmittels entsteht zwischen den beiden Schalen zudem eine Wirkverbindung. Durch die auftretende Schrumpfspannung wird das Formschlussmittel auf ein entsprechendes Mittel auf der anderen Schale gedrückt. Die Schrumpfspannung bewirkt, dass eine dichtende Wirkverbindung entsteht.

Beim erfindungsgemässen Verfahren kann ein zusätzliches Dichtmittel durch die Schrumpfspannung zwischen den formschlüssigen Mitteln der Aussenschale und der Mittel der Innenschale eingelegt oder aufgetragen werden, welches durch die Schrumpfspannung dichtend zwischen die formschlüssige Mittel der Aussenschale und der Mittel der Innenschale gepresst wird. Bevorzugt ist das zusätzliche Dichtmittel dabei ein Dichtungsring, beispielsweise aus Kunststoff oder einem metallischen Werkstoff, sowie auch eine Dichtmasse aus Kunststoff, Metall oder Keramik. Durch das zusätzliche Dichtmittel kann die Abdichtung des Zwischenraumes zwischen Innen- und Aussenschale zusätzlich verbessert werden.

Bevorzugt wird die Aussenschale beim erfindungsgemässen Verfahren zunächst erhitzt, wobei sich die Schale ausdehnt. Danach wird die erhitzte Aussenschale auf eine vorzugsweise keramische Innenschale gesetzt. Beim anschliessende Erkalten schrumpft die Aussenschale auf die Innenschale, wobei diese kraftschlüssig miteinander verbunden werden. Die Aussenschale wird dabei bevorzugt auf eine Temperatur von 100°C bis 800°C erhitzt.

Alternativ kann beim erfindungsgemässen Verfahren auch die Innenschale gekühlt werden, bevorzugt auf eine Temperatur von - 100°C bis -196°C. Die Kühlung kann beispielsweise durch Verwendung von flüssigem Stickstoff erreicht werden. Durch die Kälte zieht sich die Innenschale zusammen. Anschliessend kann diese in die Aussenschale eingesetzt werden. Beim anschliessenden Aufwärmen dehnt sich die Innenschale in die Aussenschale ein, wodurch diese kraftschlüssig miteinander verbunden werden.

Weitere Vorteile und Einzelmerkmale der Erfindung ergeben sich aus der nachfolgenden Beschreibung von Ausführungsbeispielen und aus den Zeichnungen. Es zeigen:
Fig. 1: Einen Querschnitt durch eine beispielhafte, nicht erfindungsgemässe Gelenkpfanne;
Fig. 2a - 2f: vergrösserte Darstellungen von verschiedenen beispielhaften, nicht erfindungsgemässen Ausführungsformen der Formschlussmittel im Querschnitt;
Fig. 3a-3c: Schnitte durch erfindungsgemässe Ausführungsformen einer Gelenkpfanne, bei denen die Aussenschale einen Kragen aufweist;
Fig. 4a-b: vergrösserte Schnitte von Formschlussmitteln mit zusätzlichem Dichtungsmittel; und
Fig. 5: einen Schnitt durch eine weitere Variante einer Gelenkspfanne mit zusätzlichem Dichtungsmittel.

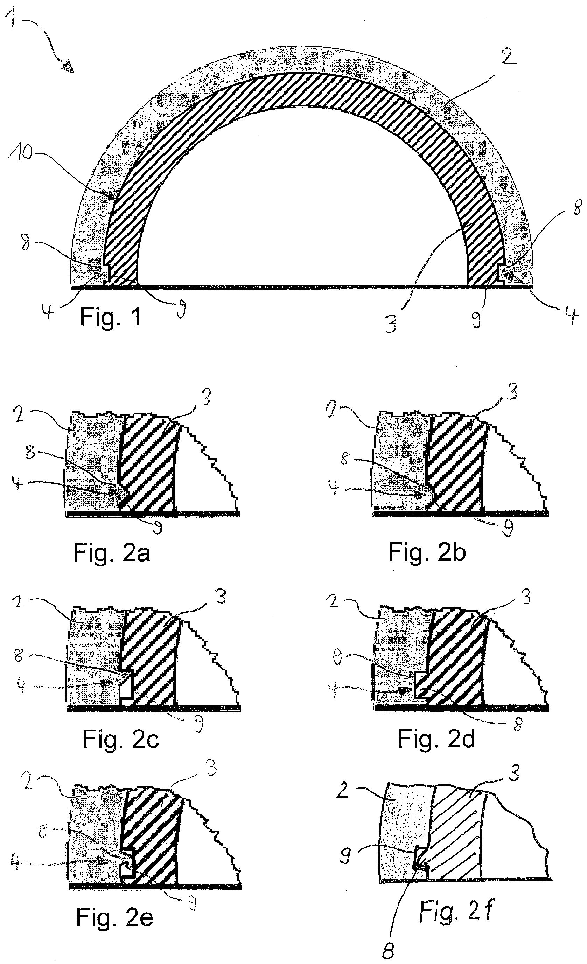

Figur 1 zeigt eine beispielhafte, nicht erfindungsgemässe Gelenkpfanne 1 im Schnitt. Die Gelenkpfanne 1 besteht aus einer metallischen Aussenschale 2 welche auf eine bevorzugt keramische Innenschale 3 aufgeschrumpft ist. Durch das Aufschrumpfen werden die beiden Schalen kraftschlüssig zusammengehalten. Zusätzliche umlaufende Formschlussmittel 4 ermöglichen eine Wirkverbindung zwischen der Aussenschale 2 und der Innenschale 3. Die Formschlussmittel 4 sind im äquatorialen Randbereich der Gelenkpfanne angebracht. Bei dieser Ausführungsform bestehen die Formschlussmittel aus einer umlaufenden Rippe 8 auf der Innenseite der Aussenschale 2 sowie einer umlaufenden Nut 9 auf der Aussenseite der Innenschale 3. Durch die Schrumpfspannung hat die Wirkverbindung zusätzlich eine Dichtwirkung. Dadurch ist die Kontaktfläche 10 zwischen der Innenschale 3 und der Aussenschale 2 gegen Aussen und Innen hin abgedichtet. Dies verhindert das Eindringen von Körperflüssigkeiten auf die Kontaktfläche 10. Ausserdem wird verhindert, dass Partikel, die bei beim Einsetzen der Gelenkspfanne oder bei der Benutzung des Gelenks durch Abrieb an der Kontaktfläche 10 entstehen, in den Körper austreten können.

Figur 2 zeigt alternative Ausführungsformen der Formschlussmittel 4, welche aus jeweils einer umlaufenden Rippe 8 sowie einer umlaufenden Nut 9 bestehen. Bei der in Figur 2a gezeigten Ausführungsform hat die Rippe 8 auf der Innenseite der Aussenschale 2 ein dreieckiges Profil. Die Nut 9 auf der Aussenseite der Innenschale 3 weist ebenfalls ein dreieckiges Profil auf und ist derart dimensioniert und angeordnet, dass die Rippe 8 passgenau oder mit Übermass in sie eingreifen kann. Figur 2b zeigt eine Ausführungsform, bei der die umlaufende Rippe 8 sowie die Nut 9 ein rundes Profil aufweisen. Bei der in der Figur 2c gezeigten Ausführungsform weisen die umlaufende Rippe 8 sowie die Nut 9 unterschiedliche Profile auf. Die Rippe 8 weist dabei ein keilförmiges Profil auf, während die Nut 9 ein viereckiges Profil aufweist. Die keilförmige Rippe 8 ist dabei derart dimensioniert und angeordnet, dass sich beim Aufschrumpfen eine Seitenfläche bündig an eine Seitenfläche der Nut 9 anlegt. Die Figur 2d zeigt eine weitere Ausführungsform der Formschlussmittel 4, bei der die Rippe 8 umlaufend auf der Innenschale 3 liegt. Die Rippe 8 greift dabei in eine auf der Innenseite der Aussenschale 2 umlaufend angebrachte Nut 9 ein. Die Figur 2e zeigt eine weitere alternative Ausführungsform des formschlüssigen Mittels 4, bei der die Rippe 8 aufgrund der Schrumpfspannung an die Aussenfläche der Nut 9 gedrückt wird, wobei sich die Rippe 8 verformt. Bei dieser Ausführungsform besteht die Schale, an der die Rippe 8 angebracht ist, bevorzugt aus einem Werkstoff mit einem geringeren Härtegrad als der Werkstoff der Schale, auf der die Nut 9 angeordnet ist.

Figur 2f zeigt eine Variante, bei der die Aussenschale 2 ebenfalls aus weicherem Material besteht als die Innenschale 3. Die Rippe 8 rastet zuerst in die Nut 9 der Aussenschale ein und presst sich dann in den Nutengrund.

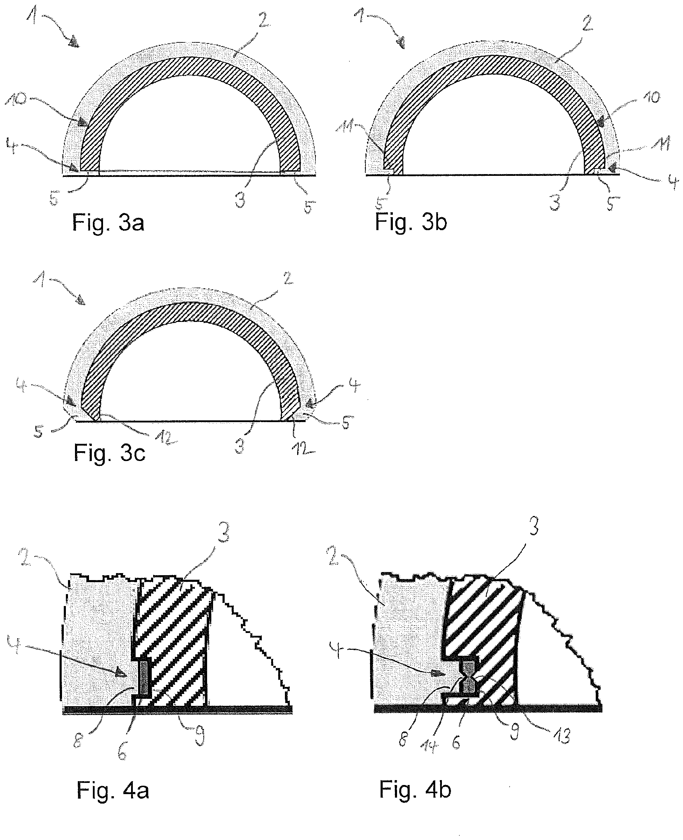

Figur 3a zeigt eine Ausführungsform einer erfindungsgemässen Gelenkpfanne 1. Dabei weist die Aussenschale 2 einen umlaufenden Kragen 5 auf, der über den unteren Rand der Innenschale 3 greift. Dies ermöglicht einen besonders guten Sitz der Aussenschale 2 auf der Innenschale 3. Die Dichtwirkung entsteht dabei durch das Anpressen des Kragens 5 an die Unterseite der Innenschale 3. Die Figur 3b zeigt eine alternative Ausführungsform, bei der der Kragen 5 in eine am unteren Rand der Innenschale angeordnete Aussparung 11 eingreift. Diese Ausführungsform der formschlüssigen Mittel reduziert den Abrieb an der Kontaktfläche 10, da Mikrobewegungen der beiden Schalen 2,3 gegeneinander verhindert werden. Ausserdem wird eine Krafteinwirkung auf die Aussenschale 2 beim Implantieren der Gelenkspfanne 1 vermieden. Die Figur 3c zeigt eine weitere alternative Ausführungsform, bei der der umlaufende Kragen 5 der Aussenschale 2 an eine schräg verlaufende Kante 12 der Innenschale 3 greift.

Die Figur 4 zeigt alternative Ausführungsformen der Formschlussmittel 4 im Schnittbild und in vergrösserter Darstellung. Bei der Ausführungsform der Figur 4a ist zwischen Rippe 8 und Nut 9 ein zusätzliches Dichtelement 6 angeordnet. Durch die Schrumpfspannung wird die Rippe 8 in das zusätzliche Dichtelement 6 gedrückt, wodurch eine besonders gute Dichtwirkung erreicht wird. Das zusätzliche Dichtmittel 6 ist dabei vorzugsweise als Dichtring ausgebildet. Die Figur 4b zeigt eine alternative Ausführungsform, bei der die Aussenschale 2 sowie die Innenschale 3 jeweils eine Lippe 13, 14 aufweisen, welche in das zusätzliche Dichtelement 6 gedrückt werden. Eine solche Anordnung erhöht zusätzlich den Dichteffekt der formschlüssigen Mittel 4.

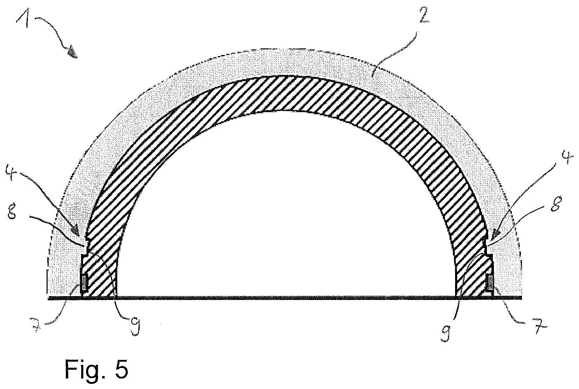

Die Figur 5 zeigt eine weitere Variante einer Gelenkpfanne 1. Bei dieser Variante ist ein zusätzliches Dichtmittel 7 zwischen der Aussenschale 2 und der Innenschale 3 angeordnet. Das zusätzliche Dichtmittel 7 befindet sich ausserhalb der Formschlussmittel 4. Beispielsweise kann das zusätzliche Dichtmittel 7 als Dichtring ausgebildet sein. Vorzugsweise weisen die Aussenschale 2 sowie die Innenschale 3 jeweils eine umlaufende Nut auf, die zur Aufnahme des zusätzlichen Dichtmittels 7 geeignet ist.

## Patentansprüche

1. Gelenkpfanne für eine Hüftgelenksprothese, mit einer vorzugsweise metallischen Aussenschale (2) und mit einer vorzugsweise keramischen Innenschale (3), wobei die Aussenschale (2) und die Innenschale (3) mittels Schrumpfsitz kraftschlüssig zusammengehalten und durch mindestens ein zusätzliches formschlüssiges Mittel (4;5) miteinander verbunden sind, wobei das mindestens eine formschlüssige Mittel (4;5) im äquatorialen Randbereich der Gelenkpfanne derart umlaufend angeordnet ist, dass dieses durch die Schrumpfspannung eine dichtende Wirkverbindung zwischen Aussenschale (2) und Innenschale (3) bildet, **dadurch gekennzeichnet, dass** die Aussenschale (2) einen umlaufenden Kragen (5) aufweist, welcher über den unteren Rand der Innenschale (3) greift.

2. Gelenkpfanne nach Anspruch 1, **dadurch gekennzeichnet, dass** das formschlüssige Mittel (4;5) mindestens eine umlaufende Rippe (8), welche bevorzugt in eine umlaufende Nut (9) eingreift, umfasst.

3. Gelenkpfanne nach Anspruch 1 oder 2, **dadurch gekennzeichnet, dass** die Gelenkpfanne (1) zwischen der Aussen- (2) und der Innenschale (3) mindestens ein zusätzliches, umlaufendes Dichtelement (7), bevorzugt einen Dichtring, aufweist.

4. Gelenkpfanne nach einem der Ansprüche 1 bis 3, **dadurch gekennzeichnet, dass** die Aussenschale (2) aus Titan, einer Titan-, Cobalt-Chrom oder Eisenbasislegierung besteht.

5. Gelenkpfanne nach einem der Ansprüche 1 bis 4, **dadurch gekennzeichnet, dass** die Innenschale (3) aus einem keramischen Werkstoff besteht, bevorzugt aus der Gruppe Aluminiumoxidkeramik, Zirkonoxidkeramik, Aluminium-Zirkon-Mischkeramiken, aluminium-toughened airconia, zirconium toughened aluminia, sowie Karbid- und Nitridkeramiken.

6. Gelenkpfanne nach einem der Ansprüche 1 bis 5, **dadurch gekennzeichnet, dass** das mindestens eine formschlüssige Mittel (4;5) eine Schneidkante auf der einen Schale (2,3) umfasst, welche infolge der Schrumpfspannung in die andere Schale (2,3) gräbt.

7. Gelenkpfanne nach Anspruch 6, **dadurch gekennzeichnet, dass** die Schneidkante auf derjenigen Schale angeordnet ist, deren Material gegenüber dem Material der anderen Schale einen höheren Härtegrad aufweist.

8. Gelenkpfanne nach einem der Ansprüche 1 bis 5, **dadurch gekennzeichnet, dass** eine Rippe auf derjenigen Schale angeordnet ist, deren Material gegenüber dem Material der anderen Schale einen kleineren Härtegrad aufweist und diese Rippe durch die Schrumpfspannung deformiert wird.

9. Verfahren zur Herstellung einer Gelenkpfanne nach einem der Ansprüche 1 bis 8, **dadurch gekennzeichnet, dass** eine metallische Aussenschale (2) auf eine bevorzugt keramische Innenschale (3) derart aufgeschrumpft wird, dass eine kraftschlüssige Verbindung entsteht, wobei mindestens ein umlaufendes formschlüssiges Mittel (4;5) als umlaufender Kragen (5) im äquatorialen Randbereich der Aussenschale (2) über den unteren Rand der Innenschale (3) dichtend greift.

10. Verfahren nach Anspruch 9, **dadurch gekennzeichnet, dass** ein zusätzliches Dichtmittel (6), bevorzugt ein Dichtungsring, zwischen der Aussenschale (2) und der Innenschale (3) eingelegt wird und durch die Schrumpfspannung zwischen den formschlüssigen Mitteln (4;5) der Aussenschale (2) und der Mittel der Innenschale (3) dichtend gepresst wird.

11. Verfahren nach einem der Ansprüche 9 oder 10, **dadurch gekennzeichnet, dass** die Aussenschale (2) erhitzt wird, bevorzugt auf eine Temperatur von 100°C bis 800°C und anschliessend im erhitzten Zustand auf die Innenschale (3) gesetzt wird.

12. Verfahren nach einem der Ansprüche 9 oder 10, **dadurch gekennzeichnet, dass** die Innenschale (3) abgekühlt wird, bevorzugt auf eine Temperatur von -100°C bis -176°C und anschliessend im erkalteten Zustand in die Aussenschale (2) eingesetzt wird.

## Claims

1. Joint socket for a hip joint prosthesis, having a preferably metallic outer shell (2) and having a preferably ceramic inner shell (3), wherein the outer shell (2) and the inner shell (3) are held together in a force-fitting manner by means of a shrink fit and are connected to one another by at least one additional form-fitting means (4; 5), wherein the at least one form-fitting means (4; 5) is arranged peripherally in the equatorial edge region of the joint socket in such a manner that, by virtue of the shrinkage stress, it forms a sealing operative connection between the outer shell (2) and the inner shell (3), **characterized in that** the outer shell (2) has a peripheral collar (5), which reaches over the bottom edge of the inner shell (3).

2. Joint socket according to Claim 1, **characterized in that** the form-fitting means (4; 5) comprises at least one peripheral rib (8), which preferably engages into a peripheral groove (9).

3. Joint socket according to Claim 1 or 2, **characterized in that** the joint socket (1) has at least one additional, peripheral sealing element (7), preferably a sealing ring, between the outer shell (2) and the inner shell (3).

4. Joint socket according to one of Claims 1 to 3, **characterized in that** the outer shell (2) consists of titanium, of a titanium alloy, of a cobalt-chromium alloy or of an iron-based alloy.

5. Joint socket according to one of Claims 1 to 4, **characterized in that** the inner shell (3) consists of a ceramic material, preferably from the group consisting of aluminum oxide ceramic, zirconium oxide ceramic, aluminum-zirconium mixed ceramics, aluminum-toughened zirconia, zirconium-toughened aluminia and also carbide and nitride ceramics.

6. Joint socket according to one of Claims 1 to 5, **characterized in that** the at least one form-fitting means (4; 5) comprises a cutting edge on one shell (2, 3), which digs into the other shell (2, 3) as a consequence of the shrinkage stress.

7. Joint socket according to Claim 6, **characterized in that** the cutting edge is arranged on that shell of which the material has a higher degree of hardness than the material of the other shell.

8. Joint socket according to one of Claims 1 to 5, **characterized in that** a rib is arranged on that shell of which the material has a lower degree of hardness than the material of the other shell, and this rib is deformed by the shrinkage stress.

9. Method for producing a joint socket according to one of Claims 1 to 8, **characterized in that** a metallic outer shell (2) is shrink-fitted onto a preferably ceramic inner shell (3) in such a manner that a force-fitting connection is formed, wherein at least one peripheral form-fitting means (4; 5) as pheripheral collar (5) reaches sealingly over the bottom edge of the inner shell (3) in the equatorial edge region of the outer shell (2).

10. Method according to Claim 9, **characterized in that** an additional sealing means (6), preferably a sealing ring, is introduced between the outer shell (2) and the inner shell (3) and is pressed sealingly, by virtue of the shrinkage stress, between the form-fitting means (4; 5) of the outer shell (2) and the means of the inner shell (3).

11. Method according to either of Claims 9 and 10, **characterized in that** the outer shell (2) is heated, preferably to a temperature of 100°C to 800°C, and is then placed in the heated state onto the inner shell ().

12. Method according to either of Claims 9 and 10, **characterized in that** the inner shell (3) is cooled, preferably to a temperature of -100°C to - 176°C, and is then inserted in the cooled state into the outer shell (2).

## Revendications

1. Cavité cotyloïde pour une prothèse de l'articulation de la hanche, comprenant une coque extérieure de préférence métallique (2) et une coque intérieure de préférence céramique (3), la coque extérieure (2) et la coque intérieure (3) étant retenues ensemble par engagement par force par ajustement serré par contraction et étant raccordées l'une à l'autre par au moins un moyen supplémentaire d'engagement par correspondance de formes (4 ; 5), l'au moins un moyen d'engagement par correspondance de formes (4 ; 5) étant disposé sur la périphérie dans la région du bord équatorial de la cavité cotyloïde de telle sorte que celui-ci forme, par la contrainte de retrait, une liaison fonctionnelle hermétique entre la coque extérieure (2) et la coque intérieure (3), **caractérisée en ce que** la coque extérieure (2) présente un rebord périphérique (5) qui vient en prise par-dessus le bord inférieur de la coque intérieure (3).

2. Cavité cotyloïde selon la revendication 1, **caractérisée en ce que** le moyen d'engagement par correspondance de formes (4 ; 5) comprend au moins une nervure périphérique (8) qui vient en prise de préférence dans une rainure périphérique (9).

3. Cavité cotyloïde selon la revendication 1 ou 2, **caractérisée en ce que** la cavité cotyloïde (1) présente, entre la coque extérieure (2) et la coque intérieure (3), au moins un élément d'étanchéité périphérique supplémentaire (7), de préférence une bague d'étanchéité.

4. Cavité cotyloïde selon l'une quelconque des revendications 1 à 3, **caractérisée en ce que** la coque extérieure (2) se compose de titane, d'un alliage à base de titane, de cobalt-chrome ou de fer.

5. Cavité cotyloïde selon l'une quelconque des revendications 1 à 4, **caractérisée en ce que** la coque intérieure (3) se compose d'un matériau céramique, de préférence du groupe contenant la céramique à base d'oxyde d'aluminium, la céramique à base d'oxyde de zirconium, les céramiques à base de mélanges d'aluminium et de zirconium, d'airconia renforcée par de l'aluminium, de corindon renforcé par du zirconium, ainsi que de céramiques à base de carbures et de nitrures.

6. Cavité cotyloïde selon l'une quelconque des revendications 1 à 5, **caractérisée en ce que** l'au moins un moyen d'engagement par correspondance de formes (4 ; 5) comprend une arête de coupe sur l'une des coques (2, 3), qui mord dans l'autre coque (2, 3) sous l'effet de la contrainte de retrait.

7. Cavité cotyloïde selon la revendication 6, **caractérisée en ce que** l'arête de coupe est disposée sur la coque dont le matériau présente une plus grande dureté par rapport au matériau de l'autre coque.

8. Cavité cotyloïde selon l'une quelconque des revendications 1 à 5, **caractérisée en ce qu'**une nervure est disposée sur la coque dont le matériau présente une plus faible dureté par rapport au matériau de l'autre coque et cette nervure est déformée par la contrainte de retrait.

9. Procédé de fabrication d'une cavité cotyloïde selon l'une quelconque des revendications 1 à 8, **caractérisé en ce qu'**une coque extérieure métallique (2) est emmanchée par contraction sur une coque intérieure de préférence céramique (3) de telle sorte qu'il se produise une liaison par engagement par force, au moins un moyen périphérique d'engagement par correspondance de formes (4 ; 5) venant en prise hermétiquement en tant que rebord périphérique (5) dans la région de bord équatoriale de la coque extérieure (2) par-dessus le bord inférieur de la coque intérieure (3) .

10. Procédé selon la revendication 9, **caractérisé en ce qu'**un moyen d'étanchéité supplémentaire (6), de préférence une bague d'étanchéité, est inséré entre la coque extérieure (2) et la coque intérieure (3) et est pressé hermétiquement sous l'effet de la contrainte de retrait entre les moyens d'engagement par correspondance de formes (4 ; 5) de la coque extérieure (2) et les moyens de la coque intérieure (3).

11. Procédé selon l'une quelconque des revendications 9 ou 10, **caractérisé en ce que** la coque extérieure (2) est chauffée, de préférence à une température de 100°C à 800°C et est ensuite placée à l'état chauffé sur la coque intérieure (3).

12. Procédé selon l'une quelconque des revendications 9 ou 10, **caractérisé en ce que** la coque intérieure (3) est refroidie, de préférence à une température de -100°C à -176°C et est ensuite insérée dans la coque extérieure (2) à l'état refroidi.
